# DEMANDE DE BREVET EUROPEEN

(11) **EP 1 462 453 A1**
(43) Date de publication de la demande: **29.09.2004**
(21) Numéro de dépôt: 04290635.4
(22) Date de dépôt: 09.03.2004
(51) Int. Cl.: C07D 521/00, C07D 401/06, C07D 207/09, C07D 401/14, C07D 207/14, C07D 207/12, C07D 401/04, C07D 207/16, A61K 7/13

(54) **Dérivés de paraphénylènediamine à groupement pyrrolidinyle disubstitué et porteur d'un radical cationique et utilisation de ces dérivés pour la coloration de fibres kératiniques**

(30) Priorité: 28.03.2003 FR 0303873
(71) Demandeur: L'OREAL, 75008 Paris (FR)
(72) Inventeur: Ramos, Laure, 92340 Bourg Lareine (FR); Sabelle, Stéphane, 75005 Paris (FR)
(74) Mandataire: Fevrier, Murielle

(57) **Abrégé**

L'invention a pour objet de nouveaux dérivés de paraphénylènediamine à groupement pyrrolidinyle disubstitué et porteur d'un radical cationique, les compositions tinctoriales les contenant ainsi que le procédé de teinture de fibres kératiniques à partir de ces compositions.

La présente invention permet en particulier d'obtenir une coloration de fibres kératiniques chromatique, puissante, peu sélective et tenace.

## Description

L'invention a pour objet de nouveaux dérivés de paraphénylènediamine à groupement pyrrolidinyle disubstitué et porteur d'un radical cationique, les compositions tinctoriales les contenant ainsi que le procédé de teinture de fibres kératiniques à partir de ces compositions.

II est connu de teindre les fibres kératiniques et en particulier les cheveux humains avec des compositions tinctoriales contenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidines, des dérivés de pyridine, des dérivés de 5,6-dihydroxyindole, des dérivés de 5,6-dihydroxyindoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-hydroxyphénols et certains composés hétérocycliques tels que par exemple des dérivés de pyrazolo[1,5-b]-1,2,4,-triazoles, des dérivés de pyrazolo[3,2-c]-1,2,4,-triazoles, des dérivés de pyrazolo[1,5-a]pyrimidines, des dérivés de pyridine, des dérivés de pyrazol-5-one, des dérivés d'indoline et des dérivés d'indole.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possibles, c'est à dire permettre d'obtenir des écarts de coloration les plus faibles possibles tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

Dans le domaine de la coloration capillaire, la para-phénylènediamine, la para-toluènediamine sont des bases d'oxydation largement utilisées. Elles permettent d'obtenir avec des coupleurs d'oxydation des nuances variées.

Cependant, il existe un besoin de découvrir de nouvelles bases d'oxydation présentant un meilleur profil toxicologique que la para-phénylènediamine et la para-toluènediamine, tout en permettant de conférer aux cheveux d'excellentes propriétés d'intensité de couleur, de variété de nuances, d'uniformité de la couleur et de ténacité aux agents extérieurs

II est déjà connu d'utiliser des dérivés de paraphénylènediamine subtitués par un groupement pyrrolidinique comme base d'oxydation pour la coloration de fibres kératiniques afin de remplacer la para-phénylènediamine. Par exemple, le brevet US 5,851,237 décrit l'utilisation de dérivés 1-(4-aminophényl)pyrrolidine éventuellement substitués sur le noyau benzénique. Le brevet US 5,993,491 propose l'utilisation de dérivés de N-(4-aminophényl)-2-hydroxyméthylpyrrolidine éventuellement substitués sur le noyau benzénique et sur l'hétérocycle pyrrolidinique en position 4 par un radical hydroxy.

La demande de brevet JP 11-158048 propose des compositions contenant au moins un composé choisi parmi des dérivés de para-phénylènediamine éventuellement substitués sur le noyau benzénique et dont un des atomes d'azote est compris dans un cycle de 5 à 7 chaînons carbonés.

La demande de brevet WO-02/45675 décrit des composés paraphénylènediamine dont un des groupes amino forme un cycle pyrrolidinique substitué en position 3 par un radical alkylammonium.

Cependant, ces composés ne permettent pas de conférer aux cheveux une coloration de qualité équivalente à celle obtenue avec la para-phénylènediamine ou avec la para-toluènediamine du fait d'un manque d'intensité et d'uniformité de la couleur.

Le but de la présente invention est de développer de nouvelles compositions tinctoriales ne présentant pas les inconvénients des bases d'oxydation de la technique antérieure. En particulier, le but de l'invention est de fournir de nouvelles bases d'oxydation présentant à la fois un bon profil toxicologique et des propriétés telles que les compositions tinctoriales les contenant ne dégradent pas les fibres kératiniques tout en étant capables d'engendrer des colorations intenses dans des nuances variées, peu sélectives et particulièrement résistantes.

Ce but est atteint avec la présente invention qui a pour objet des dérivés de formule (I) 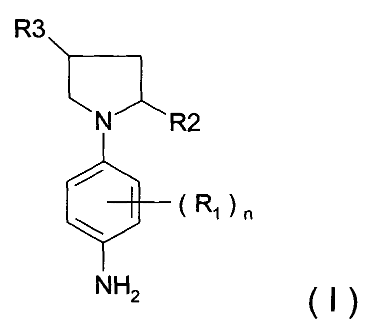 dans laquelle
- n est compris entre 0 et 4, étant entendu que lorsque n est supérieur ou égal à 2 alors les radicaux R₁ peuvent être identiques ou différents,
- R₁ représente un atome d'halogène ; un radical onium Z ; une chaîne hydrocarbonée en C₁-C₈, aliphatique ou alicyclique, saturée ou insaturée, pouvant contenir un ou plusieurs atomes d'oxygène, d'azote, de silicium, de soufre ou un groupement SO₂, la chaine hydrocarbonée pouvant être substituée par un radical hydroxyle, un radical alkyl(C1-C4)oxy, un radical amino ou un radical mono ou dialkyl(C1-C4)amino; le radical R₁ ne comportant pas de liaison peroxyde, ni de radicaux diazo, nitro ou nitroso ,
- R₂ représente un radical onium Z ; un radical carboxyle, un radical alkyl(C₁-C₄)carboxyle, un radical carbamoyle, un radical (alkyl ou dialkyl)(C₁-C₄) carbamoyle, un radical alkyle en C₁-C₆, un radical alkyle en C₁-C₆ pouvant être insaturé, substitué par un ou plusieurs radicaux hydroxy, alkyl(C₁-C₄)oxy, amino, mono- ou di-alkyl(C₁-C₄)amino, thiol, alkyl(C₁-C₄)sulfonique ou halogène, un radical alkyle en C₁-C₆ pouvant être insaturé, substitué par un ou plusieurs radicaux carboxylique, alkyl(C₁-C₄)carbonyle, alkyl(C₁-C₄)oxycarbonyle, carbamoyle, mono- ou di-alkyl(C₁-C₄)carbamoyle ; un ou plusieurs hétérocycliques azoté, oxygéné et/ou soufré, saturé et/ou insaturé à 4, 5, 6 ou 7 atomes
- R₃ représente un radical onium Z ; un atome d'hydrogène ; un radical hydroxyle; un radical alkyl(C₁-C₄)oxy ; un radical amino ; un radical mono- ou di-alkyl(C₁-C₄)amino ; un radical thiol, un radical carboxyle ; un radical alkyl(C₁-C₄)carboxyle ; un radical carbamoyle ; un radical (alkyl ou dialkyl)(C₁-C₄) carbamoyle ; un radical C₁-C₆alkylsulphonyl ; un radical alkyle en C₁-C₆ ; un radical alkyle en C₁-C₆ pouvant être insaturé, substitué par un ou plusieurs radicaux hydroxy, alkyl(C₁-C₄)oxy, amino, mono- ou di-alkyl(C₁-C₄)amino, thiol, alkyl(C₁-C₄)sulfonique ou halogène ; un radical alkyle en C₁-C₆ pouvant être insaturé, substitué par un ou plusieurs radicaux carboxylique, alkyl(C₁-C₆)carbonyle, alkyl(C₁-C₆)oxycarbonyle, carbamoyle, mono- ou di-alkyl(C₁-C₆)carbamoyle, par un ou plusieurs hétérocycliques azoté, oxygéné et/ou soufré, saturé et/ou insaturé à 4, 5, 6 ou 7 atomes ; un ou plusieurs radicaux hétérocycliques azoté, oxygéné et/ou soufré, saturé et/ou insaturé à 4, 5, 6 ou 7 atomes
avec la condition qu'au moins un des groupes R₂ et R₃ représente un radical Z.

L'invention a aussi pour objet, les composés nitro intermédiaires de la synthèse des dérivés de formule (I).

Un autre objet de l'invention est une composition tinctoriale contenant au moins un dérivé de paraphénylènediamine de formule (I) à titre de base d'oxydation.

Un autre objet de l'invention est l'utilisation de cette composition pour la teinture de fibres kératiniques et le procédé de teinture de fibres kératiniques, en particuliers les fibres kératiniques humaines telles que les cheveux mettant en oeuvre de la composition de la présente invention.

La composition de la présente invention permet en particulier d'obtenir une coloration de fibres kératiniques chromatique, puissante, peu sélective et tenace.

Dans le cadre de l'invention, une chaîne hydrocarbonée aliphatique est une chaîne linéaire ou ramifiée pouvant contenir des insaturations du type alcène ou alcyne. Une chaîne hydrocarbonée alicyclique est une chaîne ramifiée saturée ou insaturée ne contenant pas de structure cyclique aromatique.

On entend par le terme "onium" un radical quaternaire d'une base azotée.

Lorsque n est égal à 0 alors le cycle aromatique est uniquement substitué par les groupes amino en para l'un de l'autre.

Lorsque n est différent de 0, R₁ peut être par exemple un atome de chlore, un radical méthyle, éthyle, isopropyle, vinyle, allyle, méthoxyméthyle, hydroxyéthyle, 1-carboxyméthyle, 1-aminométhyle, 2-carboxyéthyle, 2-hydroxyéthyle, 3-hydroxypropyle, 1,2-dihydroxyéthyle, 1-hydroxy-2-aminoéthyle, 1-amino-2-hydroxyéthyle, 1,2-diaminoéthyle, méthoxy, éthoxy, allyloxy, 2-hydroxyéthyloxy.

Selon un mode de réalisation particulier, R₁ est choisi parmi un radical alkyle en C₁-C₄, un radical hydroxyalkyle en C₁-C₄, un radical aminoalkyle en C₁-C₄, un radical alcoxy en C₁-C₄, un radical hydroxyalcoxy en C₁-C₄. A titre d'exemple, R₁ peut être dans ce cas choisi parmi un radical méthyle, hydroxyméthyle, 2-hydroxyéthyle, 1,2-dihydroxyéthyle, méthoxy, isopropyloxy, 2-hydroxyéthoxy.

Le radical onium Z est selon un mode de réalisation particulier un radical de formule (II) 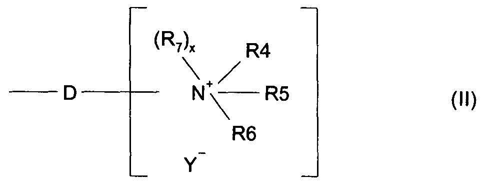 dans laquelle
- D est une liaison covalente ou une chaîne alkylène en C₁-C₁₄, linéaire ou ramifiée pouvant être interrompue par un ou plusieurs hétéroatomes choisis parmi l'oxygène, le soufre ou l'azote, et pouvant être substituée par un ou plusieurs radicaux hydroxyle, alcoxy en C₁-C₆ ou amino, et pouvant porter une ou plusieurs fonctions carbonyle ;
- R₄, R₅ et R₆, pris séparément, représentent un radical alkyle en C₁-C₁₅ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical amidoalkyle en C₁-C₆; un radical trialkyl(C₁-C₆)silanealkyle en C₁-C₆ ; un radical aminoalkyle en C₁-C₆ ; un radical aminoalkyle en C₁-C₆ dont l'amine est mono- ou di-substituée par un radical alkyle en C₁-C₆, alkyl(C₁-C₆)carbonyle, carbamoyle ou alkyl(C₁-C₆)sulfonyle ;
- R₄, R₅ et R₆ ensemble, deux à deux, forment, avec l'atome d'azote auxquels ils sont rattachés, un cycle saturé carboné à 4, 5, 6 ou 7 chaînons pouvant contenir un ou plusieurs hétéroatomes tel que par exemple un cycle azétidine, un cycle pyrrolidine, un cycle pipéridine, un cycle pipérazine ou un cycle morpholine, le cycle cationique pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical carboxyle, un radical alkyl(C₁-C₆)carbonyl, un radical thio (-SH), un radical thioalkyle (-R-SH) en C₁-C₆, un radical alkyl(C₁-C₆)thio, un radical amino, un radical amino mono ou disubstitué par un radical alkyl(C₁-C₆), alkyl(C₁-C₆)carbonyle, carbamoyle ou alkyl(C₁-C₆)sulfonyle ;
- R₇ représente un radical alkyle en C₁-C₆ ; un radical monohydroxyalkyle en C₁-C₆ ; un radical polyhydroxyalkyle en C₂-C₆ un radical aryle ; un radical benzyle ; un radical aminoalkyle en C₁-C₆ ; un radical aminoalkyle en C₁-C₆ dont l'amine est mono- ou di-subsituée par un radical alkyl(C₁-C₆), alkyl(C₁-C₆)carbonyle, carbamoyle ou alkyl(C₁-C₆)sulfonyle ; un radical carboxyalkyle en C₁-C₆; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
- x est 0 ou 1,
   - lorsque x = 0, alors le bras de liaison est rattaché à l'atome d'azote portant les radicaux R₄ à R₆,
   - lorsque x = 1, alors deux des radicaux R₄ à R₆ forment conjointement avec l'atome d'azote auquel ils sont rattachés un cycle saturé à 4, 5, 6 ou 7 chaînons et D est lié à un atome de carbone du cycle saturé ;
- Y⁻ est un contre ion.

Dans la formule (II), lorsque x est égal à 0, alors R₄, R₅ et R₆ séparément sont choisis de préférence parmi un radical alkyle en C₁-C₆, un radical monohydroxyalkyle en C₁-C₄, un radical polyhydroxyalkyle en C₂-C₄, un radical alcoxy(C₁-C₆)alkyle en C₁-C₄, un radical carbamoylealkyle en C₁-C₆, un radical trialkyl(C₁-C₆)silanealkyle en C₁-C₆ , ou R₄ avec R₅ forment ensemble un cycle azétidine, un cycle pyrrolidine, pipéridine, pipérazine, morpholine, R₆ étant choisi dans ce cas parmi un radical alkyle en C₁-C₆ ; un radical monohydroxyalkyle en C₁-C₆ ; un radical polyhydroxyalkyle en C₂-C₆; un radical aminoalkyle en C₁-C₆. un radical aminoalkyle mono ou disubstitué par un radical alkyl(C₁-C₆), alkyl(C₁-C₆)carbonyle, carbamoyle ou alkyl(C₁-C₆)sulfonyle ; un radical carbamylalkyle en C₁-C₆ ; un radical trialkyl(C₁-C₆)silanealkyle en C₁-C₆ ; un radical alkyl(C₁-C₆)carboxyalkyle en C₁-C₆ ; un radical alkyl(C₁-C₆)carbonyalkyle en C₁-C₆ ; un radical N-alkyl(C₁-C₆)carbamylalkyle en C₁-C₆ .

Lorsque x est égal à 1, alors R₇ est de préférence choisi parmi un radical alkyle en C₁-C₆ ; un radical monohydroxyalkyle en C₁-C₆ ; un radical polyhydroxyalkyle en C₂-C₆ ; un radical aminoalkyle en C₁-C₆, un radical aminoalkyle en C₁-C₆ dont l'amine est mono ou disubstituée par un radical alkyl(C₁-C₆), alkyl(C₁-C₆)carbonyle, carbamoyle ou alkyl(C₁-C₆)sulfonyle ; un radical carbamylalkyle en C₁-C₆ ; un radical trialkyl(C₁-C₆)silanealkyle en C₁-C₆ ; un radical alkyl(C₁-C₆)carboxyalkyle en C₁-C₆ ; un radical alkyl(C₁-C₆)carbonylalkyle en C₁-C₆ ; un radical N-alkyl(C₁-C₆)carbamylalkyle en C₁-C₆ ; R₄ avec R₅ ensemble forment un cycle azétidine, pyrrolidine, pipéridine, pipérazine, morpholine, R₆ étant choisi dans ce cas parmi un radical alkyle en C₁-C₆ ; un radical monohydroxyalkyle en C₁-C₆ ; un radical polyhydroxyalkyle en C₂-C₆; un radical aminoalkyle en C₁-C₆, un radical aminoalkyle en C₁-C₆ dont l'amine est mono ou disubstituée par un radical alkyl(C₁-C₆), alkyl(C₁-C₆)carbonyle, carbamoyle ou alkyl(C₁-C₆)sulfonyle; un radical carbamylalkyle en C₁-C₆ ; un radical trialkyl(C₁-C₆)silanealkyle en C₁-C₆ ; un radical alkyl(C₁-C₆)carboxyalkyle en C₁-C₆ ; un radical alkyl(C₁-C₆)carbonylalkyle en C₁-C₆ ; un radical N-alkyl(C₁-C₆)carbamylalkyle en C₁-C₆ .

Lorsque le radical R₂ correspond à la formule (II), il est de préférence un radical trialkylammonium dont les radicaux alkyles peuvent être substitués.

Dans la formule (II), D est de préférence une simple liaison ou une chaîne alkylène en C₁-C₈ pouvant être substituée.

Selon un deuxième mode de réalisation, le radical onium Z correspond à la formule (III) 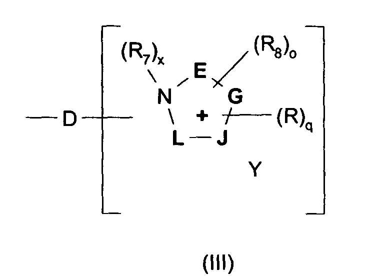 dans laquelle
- D est une liaison covalente ou une chaîne alkylène en C₁-C₁₄, linéaire ou ramifiée pouvant être interrompue par un ou plusieurs hétéroatomes choisis parmi l'oxygène, le soufre ou l'azote, et pouvant être substituée par un ou plusieurs radicaux hydroxyle, alcoxy en C₁-C₆ ou amino, et pouvant porter une ou plusieurs fonctions carbonyle ;
- les sommets E, G, J, L, identiques ou différents, représentent un atome de carbone, d'oxygène, de soufre ou d'azote pour former un cycle pyrrolique, pyrazolique, imidazolique, triazolique, oxazolique, isooxazolique, thiazolique, isothiazolique,
- q est un nombre entier compris entre 1 et 4 indus ;
- o est un nombre entier compris entre 1 et 3 inclus ;
- q+o est un nombre entier compris entre 2 et 4
- R, identiques ou différents, représentent un atome d'hydrogène, un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical carboxyle, un radical alkylcarbonyle en C₁-C₆, un radical thio, un radical thioalkyle en C₁-C₆, un radical alkyl(C₁-C₆)thio, un radical amino, un radical amino mono- ou di- substitué par un radical alkyl(C₁-C₆), alkyl(C₁-C₆)carbonyle, carbamoyle, alkyl(C₁-C₆)sulfonyle ; un radical monohydroxyalkyle en C₁-C₆ ou un radical polyhydroxyalkyle en C₂-C₆ ; étant entendu que les radicaux R sont portés par un atome de carbone,
- R₈ , identique ou différent, représente un atome d'hydrogène, un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ; étant entendu que les radicaux R₈ sont portés par un azote,
- R₇ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est substitué par un radical alkyl(C₁-C₆), alkyl(C₁-C₆)carbonyle, carbamoyle ou alkyl(C₁-C₆)sulfonyle ; un radical carboxyalkyle en C₁-C₆; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
- x est 0 ou 1
   - lorsque x = 0, le bras de liaison D est rattaché à l'atome d'azote,
   - lorsque x = 1, le bras de liaison D est rattaché à l'un des sommets E, G, J ou L,
- Y⁻ est un contre-ion.

A titre d'exemple, les sommets E, G, J et L peuvent former un cycle pyrolique, imidazolique, pyrazolique, oxazolique, thiazolique et triazolique, de préférence imidazolique.

Parmi les radicaux R₂ de formules (III), on préfère les radicaux dans lesquels x est égal à 0, D est une liaison covalente ou une chaîne alkylène en C₁-C₈ pouvant être substituée.

Selon un troisième mode de réalisation, le radical onium Z correspond à la formule (IV) 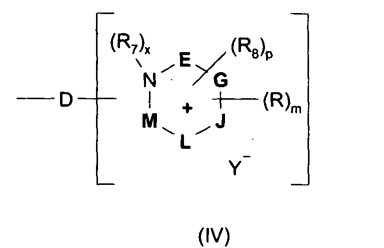 dans laquelle :
- D est une liaison covalente ou une chaîne alkylène en C₁-C₁₄, linéaire ou ramifiée pouvant être interrompue par un ou plusieurs hétéroatomes choisis parmi un atomes d'oxygène, de soufre ou d'azote, et pouvant être substituée par un ou plusieurs radicaux hydroxyle, alcoxy en C₁-C₆ ou amino, et pouvant comprendre une ou plusieurs fonctions carbonyle ;
- les sommets E, G, J, L et M identiques ou différents, représentent un atome de carbone, d'oxygène, de soufre ou d'azote former un cycle choisi parmi les cycles pyridiniques, pyrimidiniques, pyraziniques, triaziniques et pyridaziniques ;
- p est un nombre entier compris entre 1 et 3 inclus ;
- m est un nombre entier compris entre 1 et 5 inclus ;
- p+m est un nombre entier compris entre 2 et 5 ;
- R, identiques ou différents, représentent un atome d'hydrogène, un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical carboxyle, un radical alkylcarbonyle en C₁-C₆, un radical thio, un radical thioalkyle en C₁-C₆, un radical alkyl(C₁-C₆)thio, un radical amino, un radical amino substitué par un radical alkyl(C₁-C₆), alkyl(C₁-C₆)carbonyle, carbamoyle ou alkyl(C₁-C₆)sulfonyle ; un radical monohydroxyalkyle en C₁-C₆ ou un radical polyhydroxyalkyle en C₂-C₆ ; étant entendu que les radicaux R sont portés par un atome de carbone,
- R₈ , identiques ou différents, représentent un atome d'hydrogène, un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ; étant entendu que les radicaux R₈ sont portés par un azote,
- R₇ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est mono- ou di- substituée par un radical alkyl(C₁-C₆), alkyl(C₁-C₆)carbonyle, carbamoyle ou alkyl(C₁-C₆)sulfonyle ; un radical carboxyalkyle en C₁-C₆; un radical carbamylalkyle en C₁-C₆ ; un radical trifluoroalkyle en C₁-C₆ ; un radical trialkyl(C1-C6)silanealkyle en C1-C6; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
- x est 0 ou 1
   - lorsque x = 0, le bras de liaison D est rattaché à l'atome d'azote,
   - lorsque x = 1, le bras de liaison D est rattaché à l'un des sommets E, G, J, L ou M,
- Y⁻ représente un contre ion.

De préférence, les sommets E,G,J,L et M forment avec l'azote du cycle un cycle pyridinique et pyrimidinique.

Lorsque x est égal à 0 alors R est de préférence choisi parmi un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical alkylcarbonyle en C₁-C₆, un radical amino, un radical amino mono- ou di-substitué par un radical alkyl(C₁-C₆), alkyl(C₁-C₆)carbonyle, carbamoyle ou alkyl(C₁-C₆)sulfonyle ; un radical monohydroxyalkyle en C₁-C₆ ou un radical polyhydroxyalkyle en C₂-C₆ et R₈ est choisi parmi un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆.

Lorsque x est égal à 1, R₇ est de préférence choisi parmi un radical alkyle en C₁-C₆ ; un radical monohydroxyalkyle en C₁-C₆ ; un radical polyhydroxyalkyle en C₂-C₆ ; un radical aminoalkyle en C₁-C₆, un radical aminoalkyle en C₁-C₆ dont l'amine est mono- ou di- substituée par un radical alkyl(C₁-C₆), alkyl(C₁-C₆)carbonyle, un radical carbamoyle ou un radical alkyl(C₁-C₆)sulfonyle ; un radical carbamylalkyle en C₁-C₆ ; un radical trialkyl(C₁-C₆)silanealkyle en C₁-C₆ ; un radical alkyl(C₁-C₆)carbonylalkyle en C₁-C₆ ; un radical N-alkyl(C₁-C₆)carbamylalkyle en C₁-C₆ ; R est choisi parmi un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical alkylcarbonyle en C₁-C₆, un radical amino, un radical amino mono- ou di- substitué par un radical alkyl(C₁-C₆), alkyl(C₁-C₆)carbonyle, carbamoyle ou alkyl(C₁-C₆)sulfonyle ; et R₈ est choisi parmi un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆.

De préférence, R et R₈ sont des atomes d'hydrogène, des radicaux alkyles pouvant être substitués, par exemple par un ou plusieurs radicaux hydroxy, amino, aminoalkyle, alcoxy. R₇ est de préférence un radical alkyle pouvant être substitué.

Selon un mode de réalisation particulier, un seul des groupes R₂ ou R₃ représente Z.

Selon un premier mode de réalisation particulier, R₂ est un radical onium Z et R₃ est choisi parmi un atome d'hydrogène, un radical hydroxyle, un radical amino, un radical amino mono- ou di- substituée par un radical alkyl(C₁-C₆), alkyl(C₁-C₆)carbonyle, un radical carbamoyle ou un radical alkyl(C₁-C₆)sulfonyle.

Selon un second mode de réalisation particulier, R3 est un radical onium Z et R2 est choisi parmi un radical hydroxyalkyle en C₁-C₄, un radical carbamoyle, un radical mono ou dialkyl(C₁-C₄)carbamoyle, un radical carboxyle, un radical alkyl(C₁-C₄)oxycarbonyle, un radical aminoalkyle en C₁-C₄, un radical aminoalkyle en C₁-C₄ dont l'amine est mono ou disubstituée par un radical alkyle en C₁-C₄.

Selon un autre mode de réalisation, R2 et R3 sont des radicaux oniums Z.

Dans le cadre de l'invention, le contre ion Y peut être choisi parmi un atome d'halogène tel que le brome, le chlore, le fluor ou l'iode, un hydroxyde, un citrate, un succinate, un tartrate, un lactate, un tosylate, un mésylate, un benzènesulfonate, un acétate, un hydrogènesulfate ou un alkylsulfate en C₁-C₆ tel que par exemple le méthylsulfate, ou l'éthylsulfate.

A titre d'exemples de dérivés de formule (I), on peut citer :

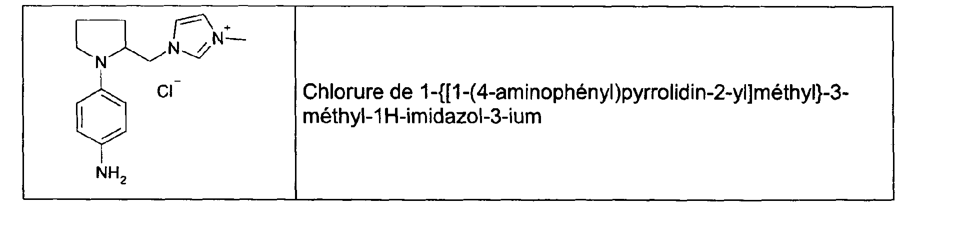

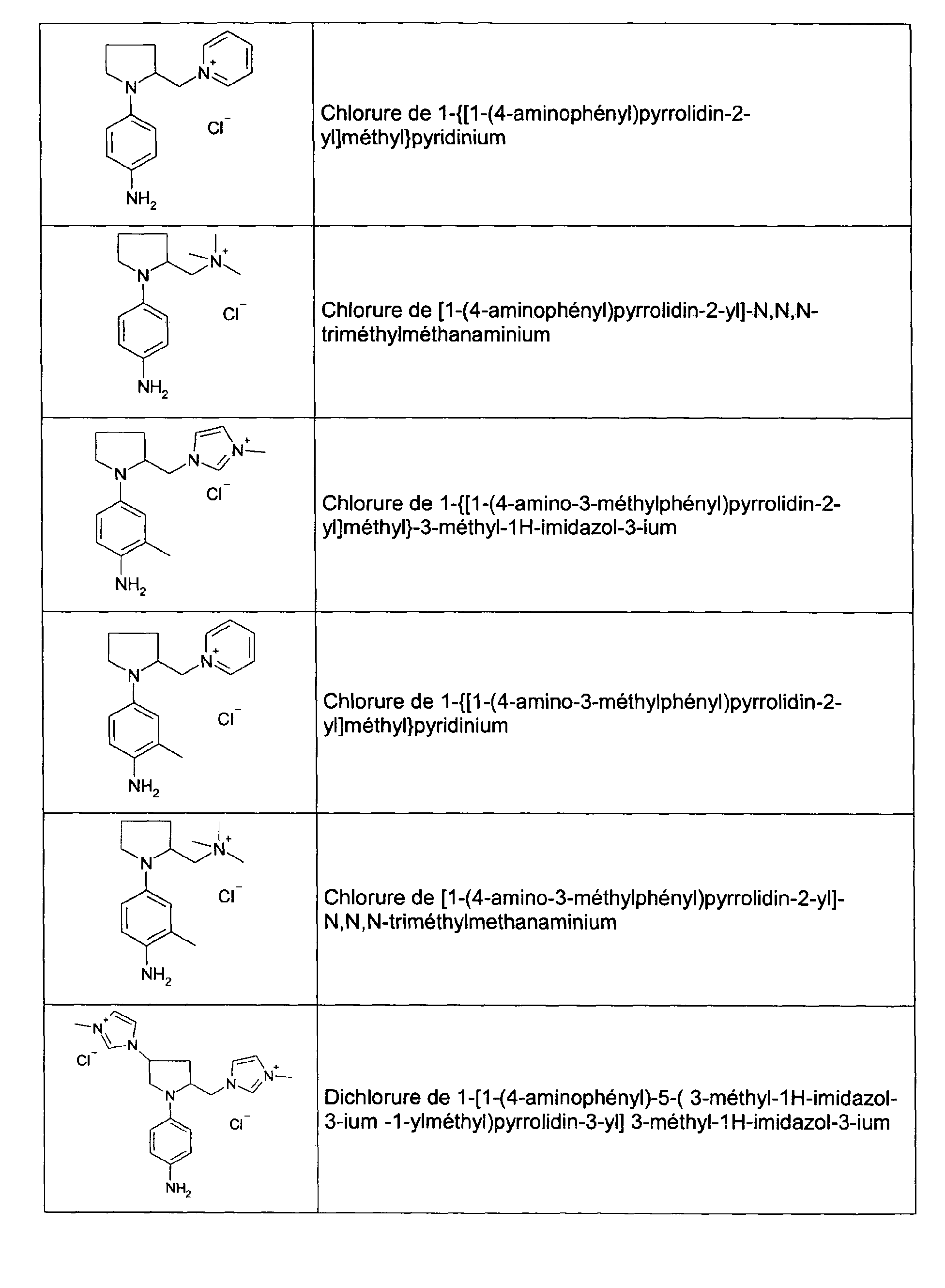

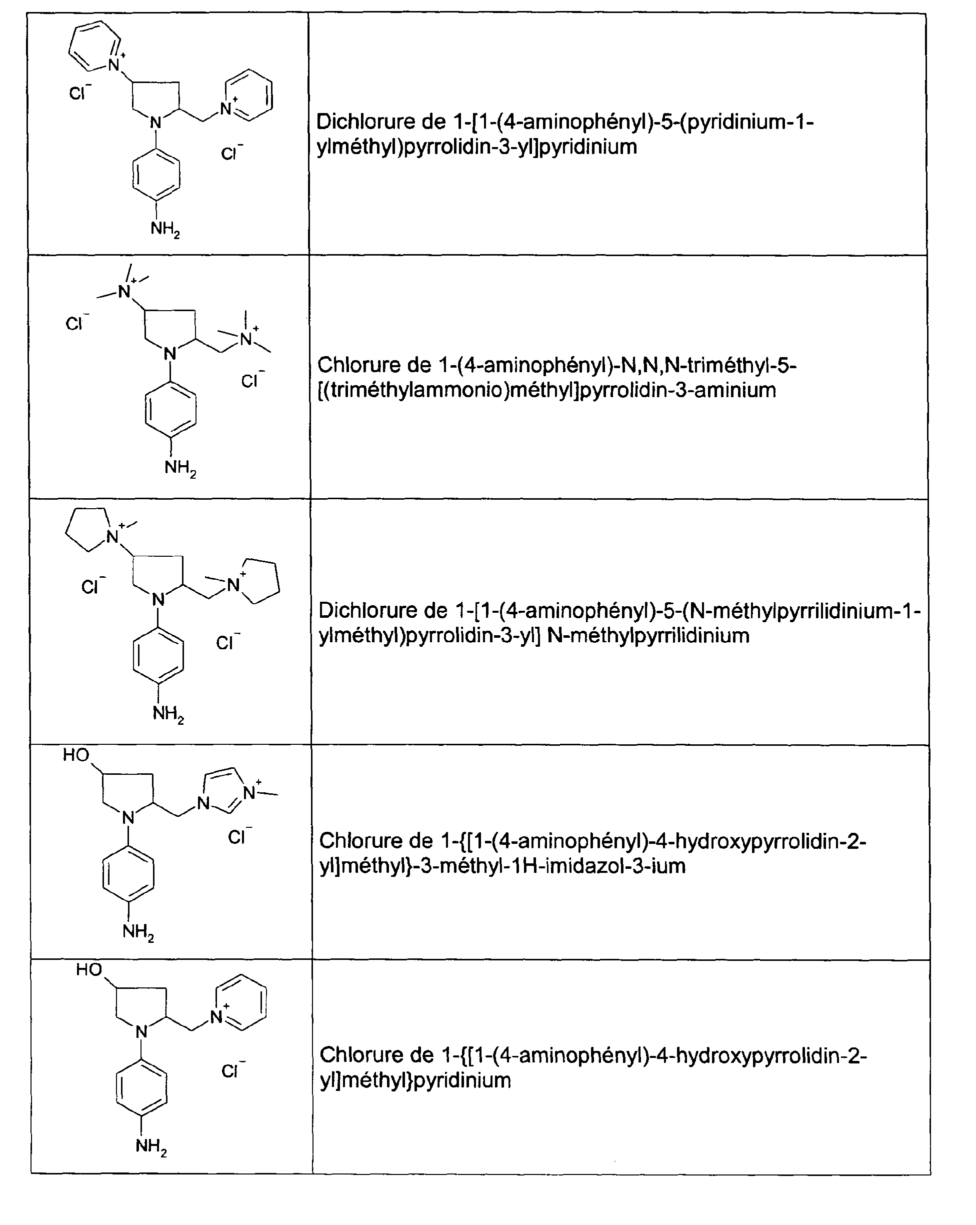

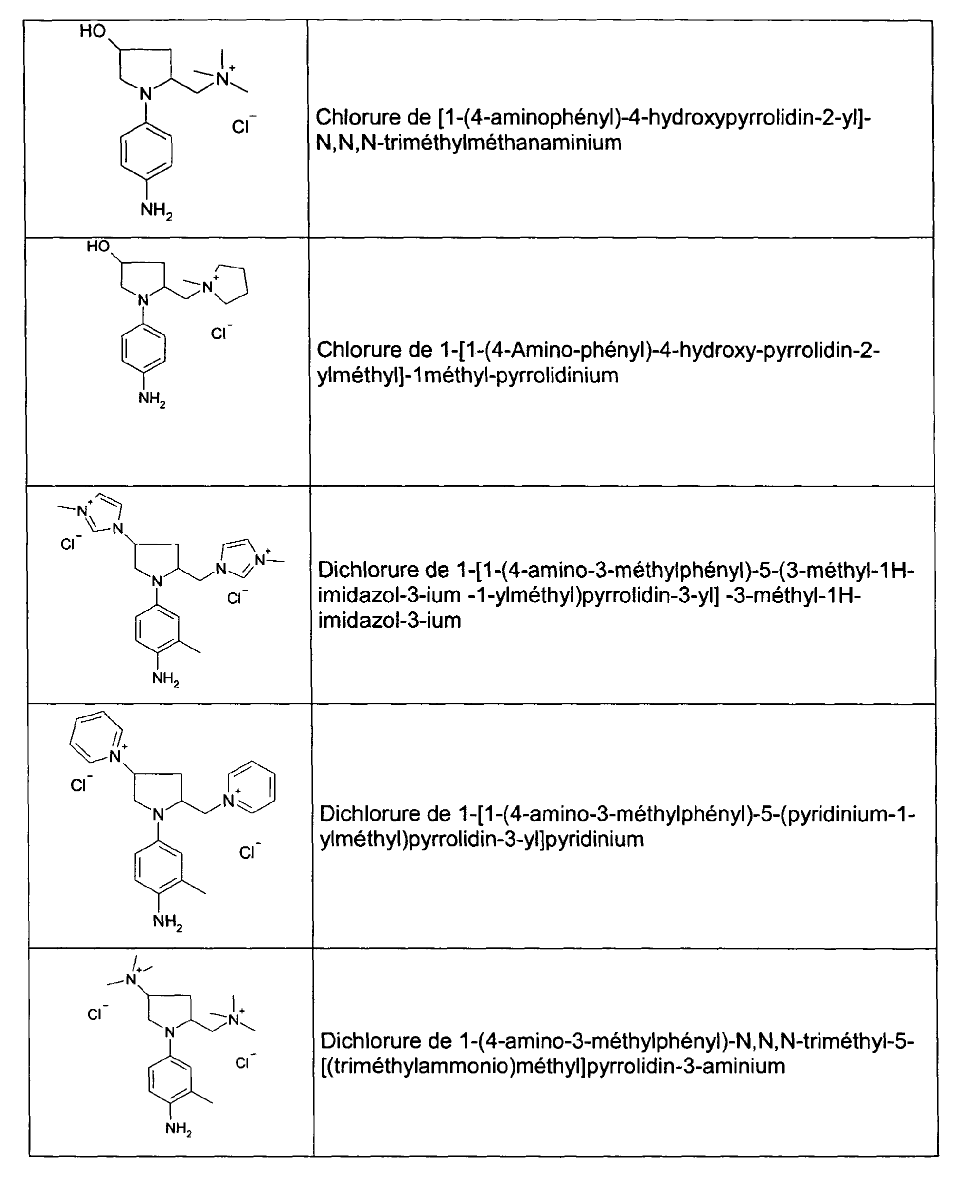

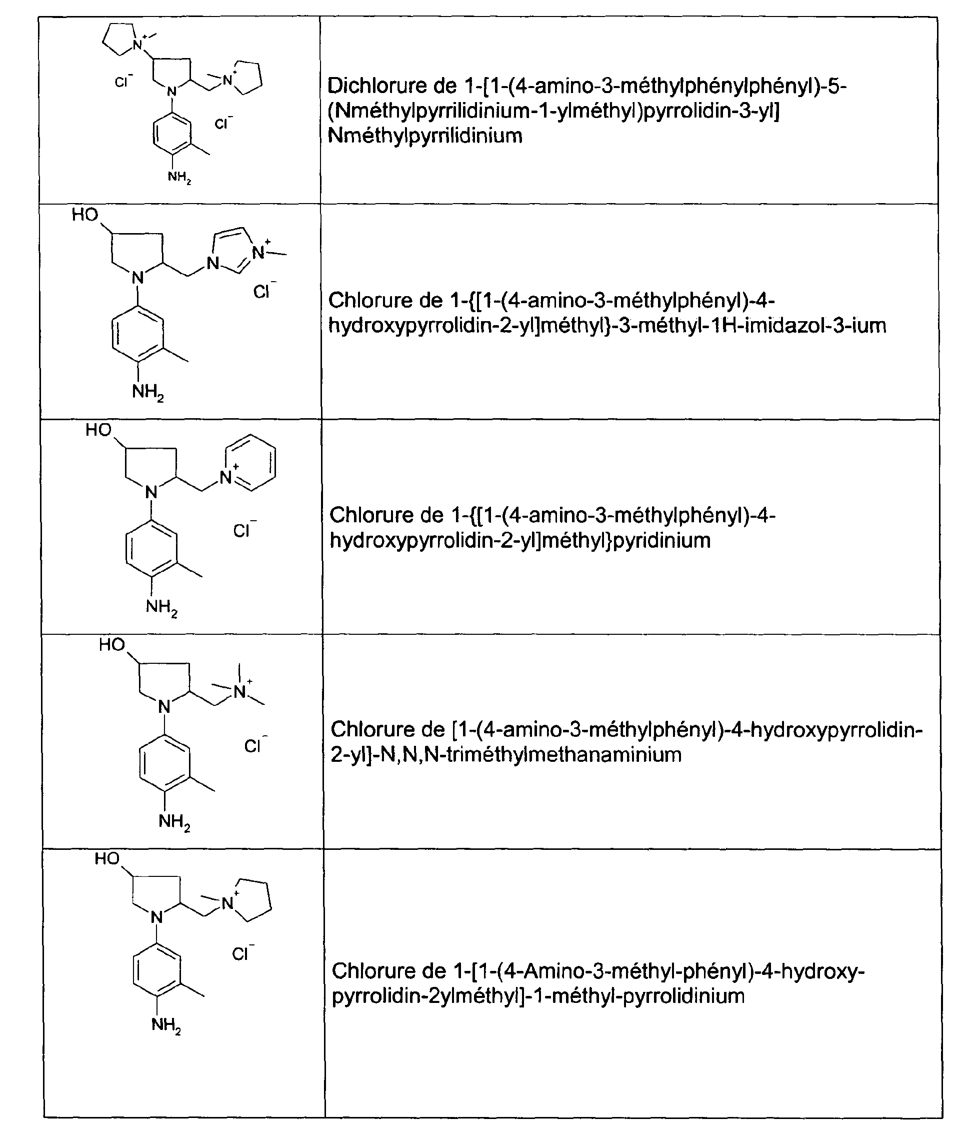

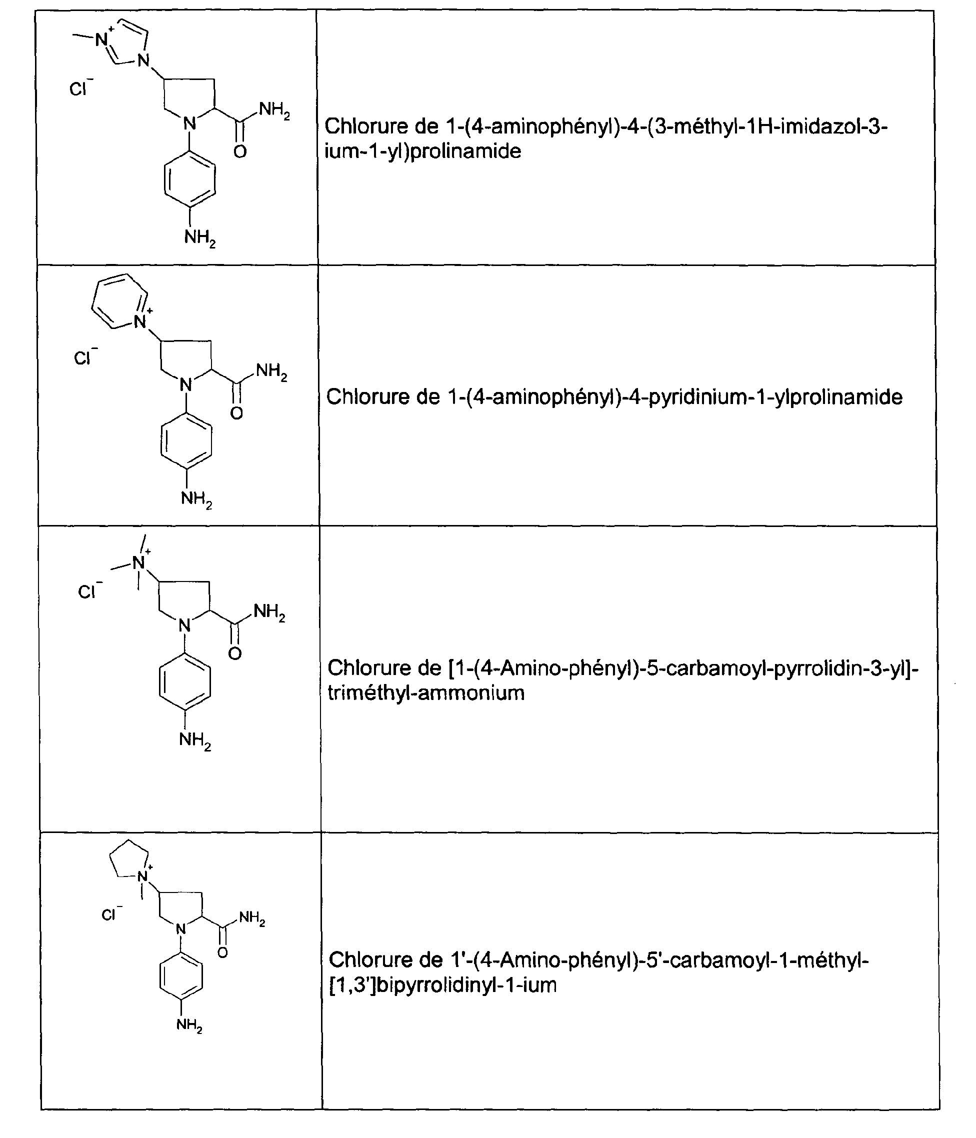

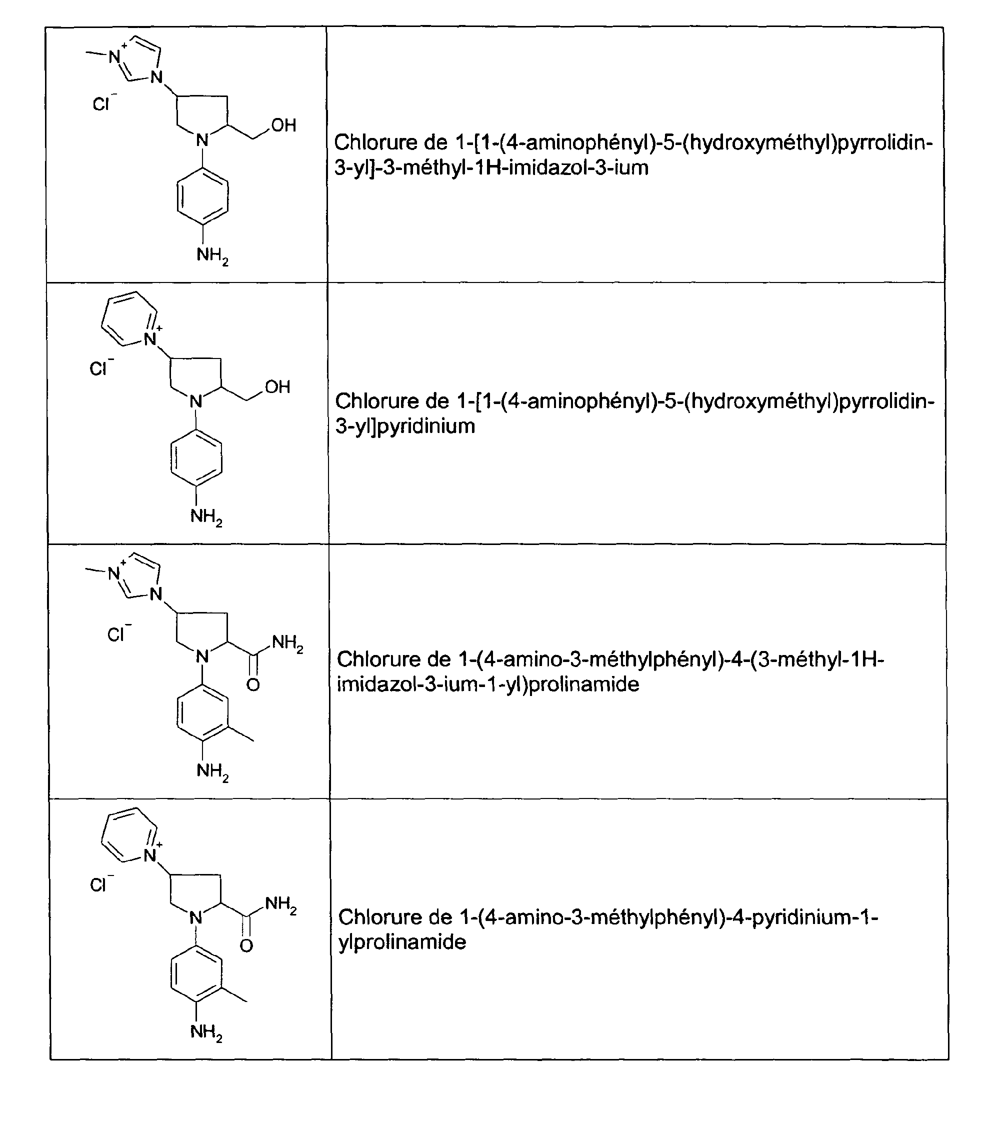

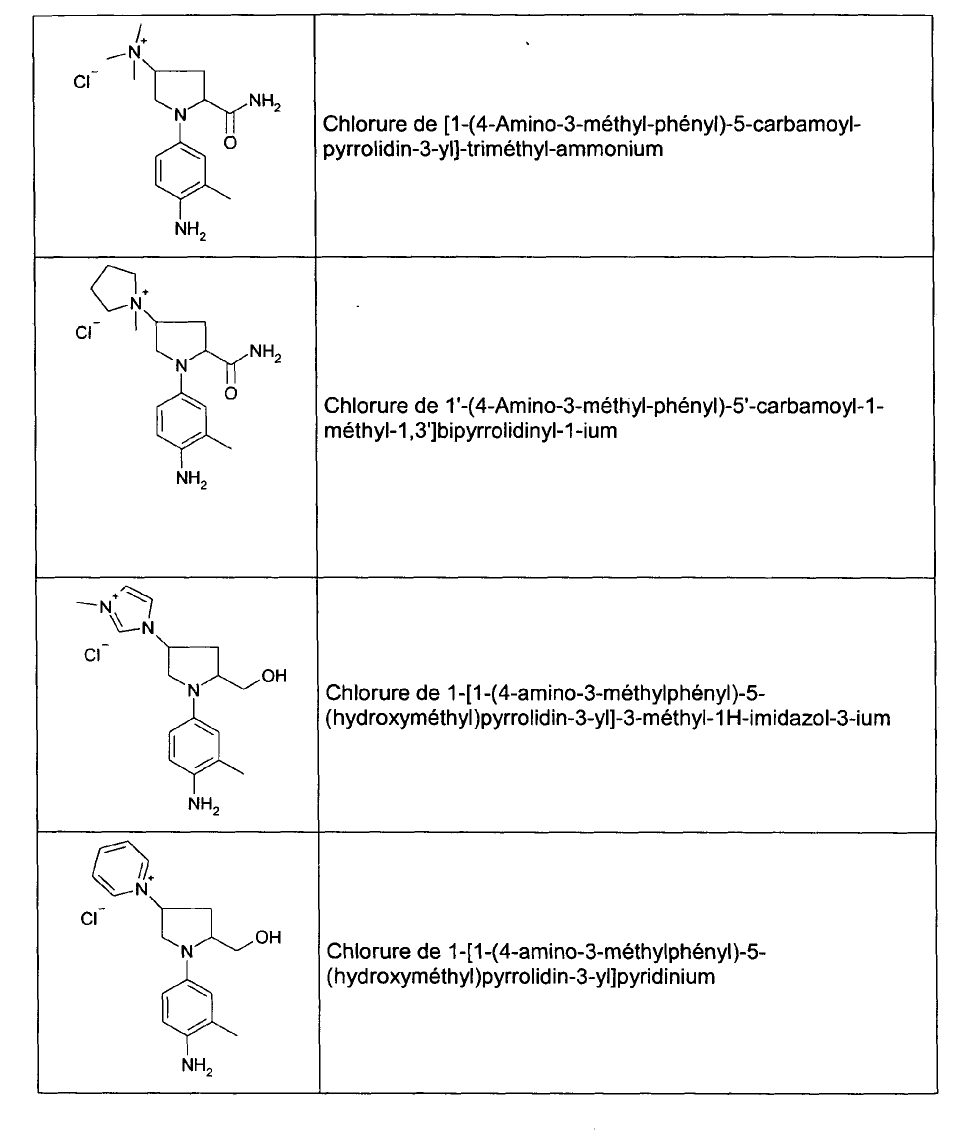

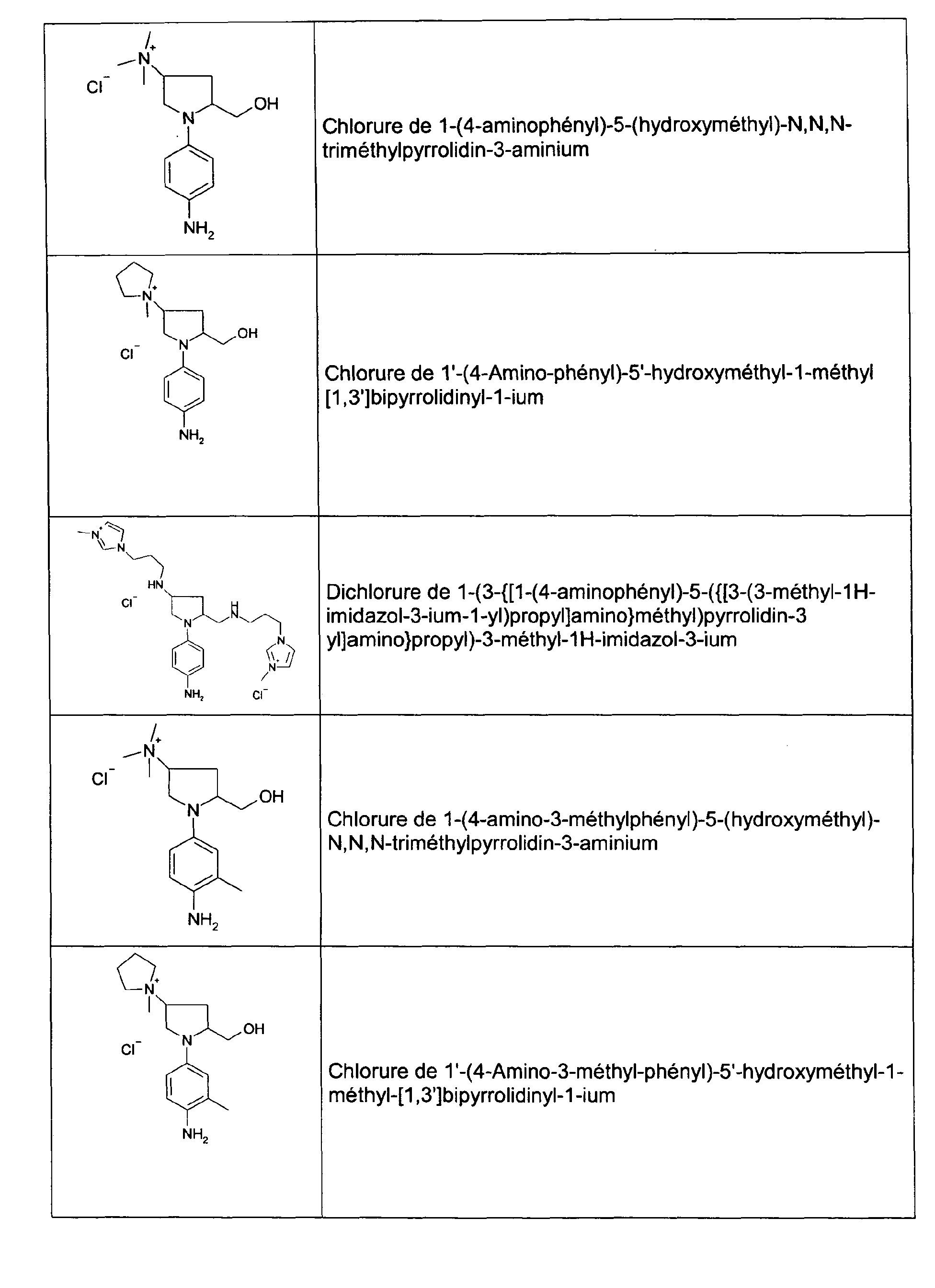

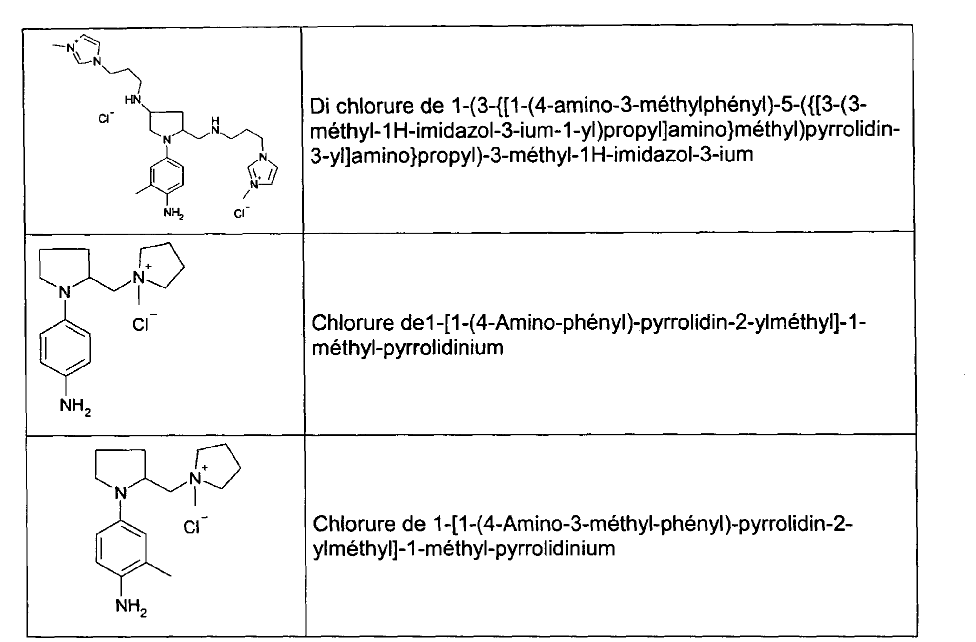

De préférence, les dérivés de formule (I) sont de préférence choisis parmi les composés suivants : Chlorure de 1-{[1-(4-aminophényl)pyrrolidin-2-yl]méthyl}-3-méthyl-1H-imidazol-3-ium, Chlorure de 1-{[1-(4-aminophényl)pyrrolidin-2-yl]méthyl}pyridinium, Chlorure de [1-(4-aminophényl)pyrrolidin-2-yl]-N,N,N-triméthylmethanaminium, Dichlorure de 1-[1-(4-aminophényl)-5-( 3-méthyl-1H-imidazol-3-ium -1-ylméthyl)pyrrolidin-3-yl] 3-méthyl-1H-imidazol-3-ium, Chlorure de 1-(4-aminophényl)-N,N,N-triméthyl-5-[(triméthylammonio)méthyl]pyrrolidin-3-aminium, Dichlorure de 1-[1-(4-aminophényl)-5-(pyridinium-1-ylméthyl)pyrrolidin-3-yl]pyridinium, Chlorure de 1-{[1-(4-aminophényl)-4-hydroxypyrrolidin-2-yl]méthyl}-3-méthyl-1H-imidazol-3-ium, Chlorure de 1-{[1-(4-aminophényl)-4-hydroxypyrrolidin-2-yl]méthyl}pyridinium, Chlorure de [1-(4-aminophényl)-4-hydroxypyrrolidin-2-yl]-N,N,N-triméthylméthanaminium, chlorure de 1-[1-(4-Amino-phényl)-4-hydroxy-pyrrolidin-2-ylméthyl]-1 méthyl-pyrrolidinium, Chlorure de 1-(4-aminophényl)-4-(3-méthyl-1H-imidazol-3-ium-1-yl)prolinamide, Chlorure de 1-(4-aminophényl)-4-pyridinium-1-ylprolinamide, Chlorure de [1-(4-Amino-phényl)-5-carbamoyl-pyrrolidin-3-yl]-triméthylammonium, chlorure de 1'-(4-Amino-phényl)-5'-carbamoyl-1-méthyl-[1,3']bipyrrolidinyl-1-ium, Chlorure de 1-[1-(4-aminophényl)-5-(hydroxyméthyl)pyrrolidin-3-yl]-3-méthyl-1H-imidazol-3-ium, chlorure de 1'-(4-Amino-phényl)-5'-hydroxyméthyl-1-méthyl[1,3']bipyrrolidinyl-1-ium, Dichlorure de 1-(3-{[1-(4-aminophényl)-5-({[3-(3-méthyl-1H-imidazol-3-ium-1-yl)propyl]amino}méthyl)pyrrolidin-3 yl]amino}propyl)-3-méthyl-1H-imidazol-3-ium, Chlorure de1-[1-( 4-Amino-phényl)-pyrrolidin-2-ylméthyl]-1-méthyl-pyrrolidinium, Dichlorure de 1-[1-(4-aminophényl)-5-(N-méthylpyrrilidinium-1-ylméthyl)pyrrolidin-3-yl] N-méthylpyrrilidinium.

Les dérivés de formule (I) peuvent par exemple être obtenus par le procédé de synthèse ci-dessous, le dérivé (1) provenant d'un mode opératoire décrit dans le brevet FR2817478 :

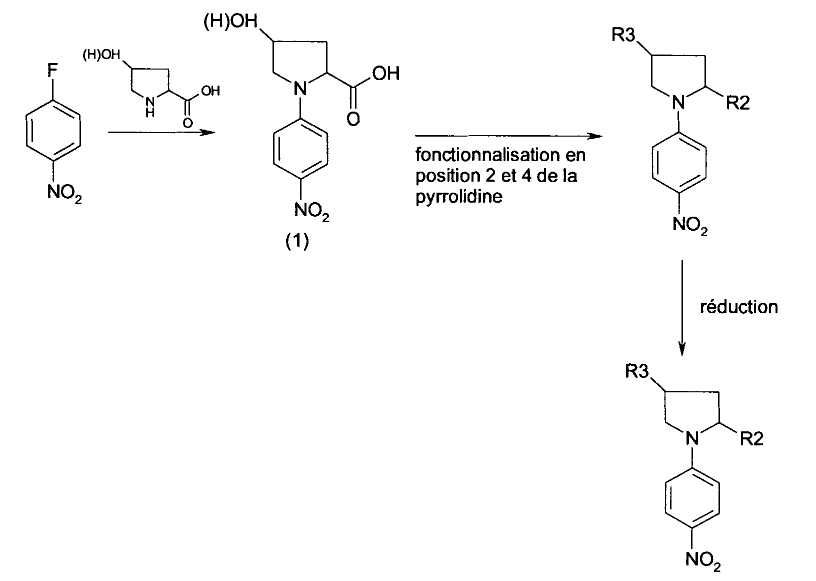

L'invention a aussi pour objet les dérivés nitro de formule (I') suivante 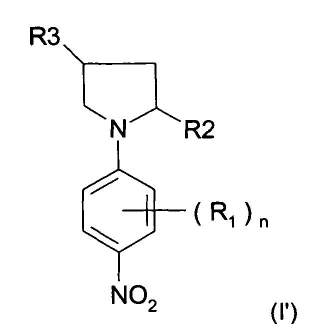 dans laquelle R₁, R₂, R₃ et n sont tels que définis précédemment

L'invention a aussi pour objet composition pour la teinture des fibres kératiniques. La composition tinctoriale de la présente invention comprend, dans un milieu approprié pour la teinture des fibres kératiniques, en particulier les cheveux humains, à titre de base d'oxydation un dérivé de formule (I) tel que défini précédemment.

La ou les bases d'oxydation de l'invention sont en général présentent chacune en quantité comprise entre 0,001 à 10 % en poids environ du poids total de la composition tinctoriale, de préférence entre 0,005 et 6 %.

La composition tinctoriale de l'invention peut contenir un ou plusieurs coupleurs conventionnellement utilisés pour la teinture de fibres kératiniques. Parmi ces coupleurs, on peut notamment citer les méta-phénylènediamines, les méta-aminophénols, les méta-diphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

La composition de la présente invention peut en outre comprendre une ou plusieurs bases d'oxydation additionnelles classiquement utilisées en teinture d'oxydation autres que celles décrites précédemment. A titre d'exemple, ces bases d'oxydation additionnelles sont choisies parmi les para-phénylènediamines autres que celles décrites précédemment, les bis-phénylalkylènediamines, les para-aminophénols, les bis-paraaminophénols, les ortho-aminophénols, les bases hétérocycliques et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl paraphénylènediamine, la N-(β-hydroxypropyl) paraphénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylène-diamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthy) para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans la composition de l'invention sont en général présentent chacune en quantité comprise entre 0,001 à 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 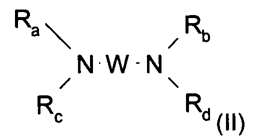 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, et on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant au moins une base d'oxydation de formule (I) avec un agent oxydant, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES

### Exemple 1 : Synthèse du Chlorure de 3-[1-(4-Amino-phényl)-pyrrolidin-2-ylméthyl]-1-méthyl-3H-imidazol-1-ium (3)

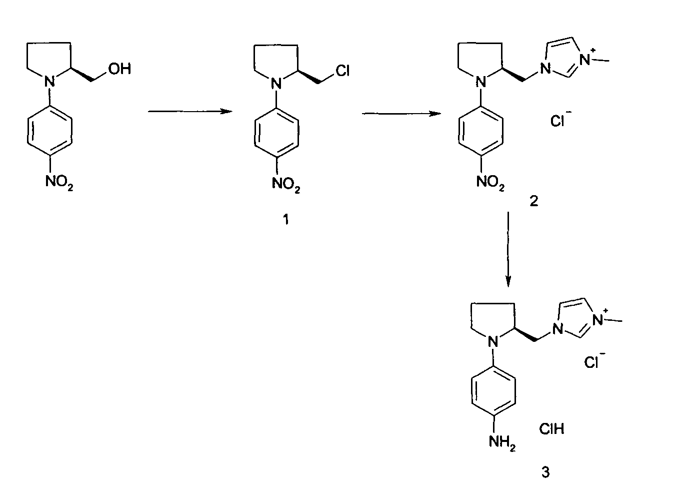

### Synthèse du 2-Chlorométhyl-1-(4-nitro-phényl)-pyrrolidine (1)

A la solution de [1-(4-Nitro-phényl)-pyrrolidin-2-yl]-méthanol (66,7g 0,03 mole), 300ml de DMF anhydre et 58,5 ml (0,42 mole) de triéthylamine anhydre, refroidie dans un bain de glace, on ajoute goutte à goutte 27,9 ml (0,36 mole) de chlorure de mésyle. On laisse remonter la température jusqu'à 20°C en une heure, sous agitation. 35,2g (0,6 mole) de chlorure de sodium sont additionnées et on chauffe une heure à 100°C. Le milieu réactionnel est versé sur 1,41 d'eau glacée. Le précipité est filtré, lavé dans l'eau, séché et recristallisé dans l'isopropanol. On obtient ainsi 63,7g d'une poudre jaune (1).
RMN 1H (400MHz-D2O) ppm 2,07 (m, 4H) ; 3,3 (m, 1H) 3,53 (m, 1H) 3,63 (m, 2H) 4,21 (m, 1H) 6,72 (m, 2H) 8,07 (m, 2H)

### Synthèse du chlorure de 1-Méthyl-3-[1-(4-nitro-phényl)-pyrrolidin-2-ylméthyl]-3H-imidazol-1-ium (2)

16,8g (0,07 mole) de 2-Chlorométhyl-1-(4-nitro-phényl)-pyrrolidine (1), 17,2g (0,21 mole) de méthylimidazole et 70ml de toluène sont chauffés au reflux pendant 9 heures. Le solvant est évaporé et le produit est cristallisé dans l'isopropanol puis séché sous vide. Obtention de 3g de cristaux jaunes (2).
RMN 1H (400MHz-D2O) ppm 1.93(m, 4H) ; 3.30(m, 1H); 3.60(m, 1H); 3.82(s, 3H); 4.30(m, 2H); 4.45(m, 1H); 6.75(d, 2H) ; 7.71(s, 1H); 7.83(d, 1H); 8.05(d, 2H); 9.25(s, 1H)

### Synthèse du chlorure de 3-[1-(4-Amino-phényl)-pyrrolidin-2-ylméthyl]-1-méthyl-3H-imidazol-1-ium; hydrochlorure (3)

3g (0,093mole) de 1-Méthyl-3-[1-(4-nitro-phényl)-pyrrolidin-2-ylméthyl]-3H-imidazol-1-ium (2) en solution dans 350 ml d'éthanol sont hydrogénés en présence de palladium sur charbon sous une pression d'hydrogène de 10 bars à une température de 30°C ; après filtration du catalyseur, le dérivé (3) attendu est isolé sous forme de chlorhydrate. On obtient 2,6g d'une poudre blanche.
RMN 1H (400MHz-D2O) ppm 1,66 (m, 1H) ; 1,89 (m, 1H) ; 2,01 (m, 1H) ; 2,13 (m, 1 H) ; 3,17 (m, 1H) ; 3,54 (m, 1H) ; 3,73 (s, 3H) ; 4,23 (dd, 1H) ; 4,33 (m ,1H) ; 4,43 (dd, 1H) ; 6,67 (d, 2H) ; 7,21 (d, 2H) ; 7,35 (s, 1H) ; 7,43 (s,1H) ; 8,59 (s,1H)

### Exemple 2 : Synthèse du dimésylate 1-[1-(4-aminophényl)-5-(3-méthyl-1H-imidazol-3-ium -1-ylméthyl)pyrrolidin-3-yl] 3-méthyl-1H-imidazol-3-ium (6)

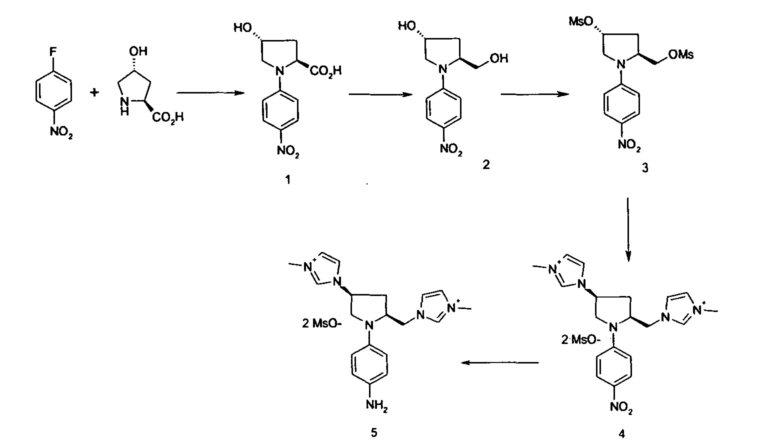

### Synthèse de l'acide 4-Hydroxy-1-(4-nitro-phenyl)-pyrrolidine-2-carboxylique (1)

10g (70.9 mmoles) de 4-fluoronitrobenzène, 11.75g (85 mmoles) de carbonate de potassium et 9.3g (70.8 mmoles) de trans-4-hydroxyproline sont agités dans 100ml d'eau distillée. On chauffe à reflux pendant 16 heures. On laisse revenir à température ambiante. Le produit est extrait à l'acétate d'éthyle. Les phases organiques sont lavées avec une solution saturée, séchées sur sulfate de sodium et concentrées sous pression réduite. L'huile obtenue est cristallisée dans un mélange acétate d'éthyle/heptane. On obtient une poudre jaune.
RMN 1H (400MHz-DMSO) ppm 1.99-2.30(m, 2H) ; 3.68(m, 1H); 4.46(m, 2H); 5.27(m, 1H); 6.58(d, 2H); 8.07(d, 2H)

### Synthèse du 5-Hydroxyméthyl-1-(4-nitro-phenyl)-pyrrolidin-3-ol (2)

5g (19.8 mmoles) de l'acide 4-Hydroxy-1-(4-nitro-phenyl)-pyrrolidine-2-carboxylique sont agités dans 50ml de THF à 3°C. 60ml(59 mmoles) du complexe borane THF sont ajoutés goutte à goutte. On agite à température ambiante pendant 5 heures. On ajoute lentement du méthanol. Le mélange réactionnel est évaporé à sec. Le produit est précipité dans une solution saturée en chlorure de sodium, filtré et séché sous vide. Obtention de 5.33g d'une poudre jaune.
RMN 1H (400MHz-DMSO) ppm 1.95(m, 1H); 2.20(m, 1H); 3.14(dd, 1H); 3.44(m, 2H); 3.63(m, 1H); 4.06(m, 1H); 4.51(m, 1H); 4.87(m, 1H); 5.12(m, 1H); 6.69(d, 2H); 8.04(d, 2H).

### Synthèse dde l'ester de 4-méthanesulfonyloxy-1-(4-nitro-phenyl)-pyrrolidin-2-ylméthyl méthane sulfonique(3)

3g (12.6 mmoles) de 5-Hydroxymethyl-1-(4-nitro-phenyl)-pyrrolidin-3-ol et 4.6ml (37.8 mmoles) de triéthylamine sont agités dans 20 ml de THF à 5°C. On ajoute lentement 2.54ml (32.8 mmoles) de chlorure de mésyle. Le mélange réactionnel est agité à température ambiante pendant 6 heures. Le produit est précipité dans de l'eau glacée et cristallisé dans le méthanol. Obtention de1.7g de poudre jaune.
RMN 1H (400MHz-DMSO) ppm 3.15(s, 3H); 3.28(s, 3H); 3.14(dd, 1H); 3.70(m, 1H); 4.90(m, 1H); 4.28-4.40(m, 2H); 4.59(m, 1H); 5.49(m, 1H); 6.87(d, 2H); 8.10(d, 2H).

### Synthèse du dimésylate 1-[1-(4-nitrophényl)-5-(3-méthyl-1H-imidazol-3-ium-1-ylméthyl)pyrrolidin-3-yl] 3-méthyl-1H-imidazol-3-ium (4)

1.7g (4.3 mmoles) de l'ester 4-methanesulfonyloxy-1-(4-nitro-phenyl)-pyrrolidin-2-ylmethyl méthane sulfonique (3) sont chauffés dans 15ml de N-méthylimidazole à 90°C pendant 25 heures. Le mélange réactionnel est refroidi. L'huile obtenue est reprise dans l'acétate d'éthyle et purifiée par HPLC préparative.

### Synthèse du dimésylate 1-[1-(4-aminophényl)-5-(3-méthyl-1H-imidazol-3-ium-1-ylméthyl)pyrrolidin-3-yl]3-méthyl-1H-imidazol-3-ium (5)

Après réduction au zinc/ acide acétique on obtient 1-[1-(4-aminophényl)-5-( 3-méthyl-1H-imidazol-3-ium-1-ylméthyl)pyrrolidin-3-yl] 3-méthyl-1H-imidazol-3-ium
Masse ESI+ : m/2z=169[M]

### Exemples de teinture

### EXEMPLES 1 A 5 DE TEINTURE EN MILIEU ALCALIN

| **Exemples** | **1** | **2** | **3** | **4** | **5** |
|---|---|---|---|---|---|
| chlorure de 3-[1-(4-Amino-phényl)-pyrrolidin-2-ylméthyl]-1-méthyl-3H-imidazol-1-ium; hydrochlorure (base) | 10⁻³ mole | 10⁻³ mole | 10⁻³ mole | 10⁻³ mole | - |
| dimésylate 1-[1-(4-aminophényl)-5-( 3-méthyl-1H-imidazol-3-ium -1-ylméthyl)pyrrolidin-3-yl] 3-méthyl-1H-imidazol-3-ium (base) | - | - | - | - | 10⁻³ mole |
| 2-(2,4-Diamino-phénoxy)-éthanol, dichlorhydrate (coupleur) | 10-3 mole | - | - | - | 10-3 mole |
| 3-Amino-2-chloro-6-méthyl-phénol, chlorhydrate (coupleur) | - | 10-3 mole | - | - | - |
| 3,6-Diméthyl-1 H-pyrazolo[5,1-c] [1,2,4]triazole (coupleur) | - | - | 10⁻³ mole | | - |
| 2-méthyl-5-aminophénol (coupleur) | - | - | - | 10⁻³ mole | - |
| Support de teinture (1) | (*) | (*) | (*) | (*) | (*) |
| Eau déminéralisée q.s.p. | 100 g | 100 g | 100 g | 100 g | 100 g |

| | | | | | |
|---|---|---|---|---|---|
| (*) Support de teinture (1) pH 9,5 | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH3 | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 min de pose, les mèches sont rinçées, lavées avec un shampooing standard, rincées à nouveau puis séchées.

Les résultats de teinture suivants ont été obtenus.

| **Exemples** | **1** | **2** | **3** | **4** | **5** |
|---|---|---|---|---|---|
| **Nuance** | Bleu | Violet | Rouge violet | violet | Gris |
| **observée** | violet | bleu | chromatique | | |

### EXEMPLES 6 A 10 DE TEINTURE EN MILIEU ACIDE

On a préparé les compositions tinctoriales suivantes :

| **Exemples** | **6** | **7** | **8** | **9** | **10** |
|---|---|---|---|---|---|
| chlorure de 3-[1-(4-Aminophényl)-pyrrolidin-2-ylméthyl]-1-méthyl-3H-imidazol-1-ium; hydrochlorure (base) | 10⁻³ mole | 10⁻³ mole | 10⁻³ mole | 10⁻³ mole | - |
| dimésylate 1-[1-(4-aminophényl)-5-( 3-méthyl-1H-imidazol-3-ium -1-ylméthyl)pyrrolidin-3-yl] 3-méthyl-1H-imidazol-3-ium (base) | - | - | - | - | 10⁻³ mole |
| 2-(2,4-Diamino-phénoxy)-éthanol, dichlorhydrate (coupleur) | 10⁻³ mole | - | - | - | 10⁻³ mole |
| 3-Amino-2-chloro-6-méthylphénol, chlorhydrate (coupleur) | - | 10⁻³ mole | - | - | - |
| 2-méthyl-5-aminophénol (coupleur) | - | - | 10⁻³ mole | - | - |
| 3,6-Diméthyl-1H-pyrazolo[5,1-c] [1,2,4]triazole (coupleur) | - | - | - | 10⁻³ mole | - |
| Support de teinture (2) | (*) | (*) | (*) | (*) | (*) |
| Eau déminéralisée q.s.p. | 100 g | 100 g | 100 g | 100 g | 100 g |

| | | | | | |
|---|---|---|---|---|---|
| (*) Support de teinture (2) pH 7 | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 min de pose, les mèches sont rincées, lavées avec un shampooing standard, rinçées à nouveau puis séchées.

Les résultats de teinture suivants ont été obtenus.

| **Exemples** | **6** | **7** | **8** | **9** | **10** |
|---|---|---|---|---|---|
| **Nuance** | Bleu | Violet | violet | Violet | gris |
| **observée** | violet | bleu | | rouge | |

## Revendications

1. Dérivés de paraphénylènediamine substitués par un groupement pyrrolidinyle de formule (I) et leurs sels d'addition 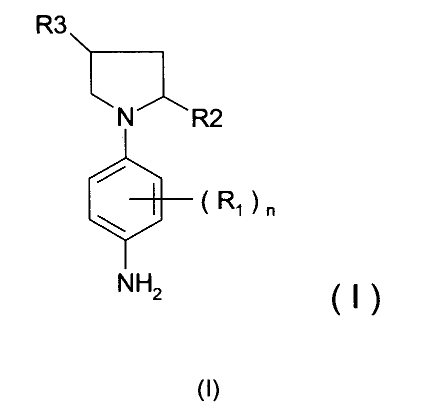 dans laquelle
• n est compris entre 0 et 4, étant entendu que lorsque n est supérieur ou égal à 2 alors les radicaux R₁ peuvent être identiques ou différents,
• R₁ représente un atome d'halogène ; un radical onium Z ; une chaîne hydrocarbonée en C₁-C₈, aliphatique ou alicyclique, saturée ou insaturée, pouvant contenir un ou plusieurs atomes d'oxygène, d'azote, de silicium, de soufre ou un groupement SO₂ , la chaine hydrocarbonée pouvant être substituée par un radical hydroxyle, un radical alkyl(C1-C4)oxy, un radical amino ou un radical mono ou dialkyl(C1-C4)amino; le radical R₁ ne comportant pas de liaison peroxyde, ni de radicaux diazo, nitro ou nitroso ,
• R₂ représente un radical onium Z ; un radical carboxyle, un radical alkyl(C₁-C₄)carboxyle, un radical carbamoyle, un radical (alkyl ou dialkyl)(C₁-C₄) carbamoyle, un radical alkyle en C₁-C₆, un radical alkyle en C₁-C₆ pouvant être insaturé, substitué par un ou plusieurs radicaux hydroxy, alkyl(C₁-C₄)oxy, amino, mono- ou di-alkyl(C₁-C₄)amino, thiol, alkyl(C₁-C₄)sulfonique ou halogène, un radical alkyle en C₁-C₆ pouvant être insaturé, substitué par un ou plusieurs radicaux carboxylique, alkyl(C₁-C₄)carbonyle, alkyl(C₁-C₄)oxycarbonyle, carbamoyle, mono- ou di-alkyl(C₁-C₄)carbamoyle ; un ou plusieurs hétérocycliques azoté, oxygéné et/ou soufré, saturé et/ou insaturé à 4, 5, 6 ou 7 atomes
• R₃ représente un radical onium Z ; un atome d'hydrogène ; un radical hydroxyle; un radical alkyl(C₁-C₄)oxy ; un radical amino ; un radical mono- ou di-alkyl(C₁-C₄)amino ; un radical thiol, un radical carboxyle ; un radical alkyl(C₁-C₄)carboxyle ; un radical carbamoyle ; un radical (alkyl ou dialkyl)(C₁-C₄) carbamoyle ; un radical (C₁-C₆)alkylsulphonyle ; un radical alkyle en C₁-C₆ ; un radical alkyle en C₁-C₆ pouvant être insaturé, substitué par un ou plusieurs radicaux hydroxy, alkyl(C₁-C₄)oxy, amino, mono- ou di-alkyl(C₁-C₄)amino, thiol, alkyl(C₁-C₄)sulfonique ou halogène ; un radical alkyle en C₁-C₆ pouvant être insaturé, substitué par un ou plusieurs radicaux carboxylique, alkyl(C₁-C₆)carbonyle, alkyl(C₁-C₆)oxycarbonyle, carbamoyle, mono- ou di-alkyl(C₁-C₆)carbamoyle, par un ou plusieurs hétérocycliques azoté, oxygéné et/ou soufré, saturé et/ou insaturé à 4, 5, 6 ou 7 atomes ; un hétérocyclique azoté, oxygéné et/ou soufré, saturé et/ou insaturé à 4, 5, 6 ou 7 atomes,
avec la condition qu'au moins un des groupes R₂ et R₃ représente un radical Z.

2. Dérivés selon la revendication 1 dans lesquels n est égal à 0 ou 1.

3. Dérivés selon la revendication 1 ou 2 dans lesquels R₁ est choisi parmi un radical alkyle en C₁-C₄, un radical hydroxyalkyle en C₁-C₄, un radical aminoalkyle en C₁-C₄, un radical alcoxy en C₁-C₄, un radical hydroxyalcoxy en C₁-C₄.

4. Dérivés selon la revendication 3 dans lesquels R₁ est choisi parmi les radicaux méthyle, hydroxyméthyle, 2-hydroxyéthyle, 1,2-dihydroxyéthyle, méthoxy, isopropyloxy, 2-hydroxyéthoxy.

5. Dérivés selon l'une quelconque des revendications 1 à 4 dans lesquels le radical onium Z correspond à la formule (II) 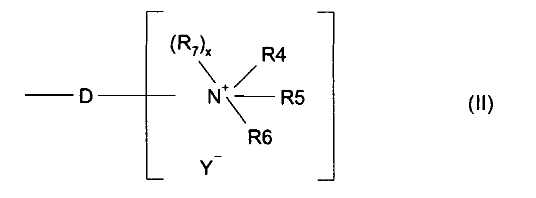 dans laquelle
• D est une liaison covalente ou une chaîne alkylène en C₁-C₁₄, linéaire ou ramifiée pouvant contenir un ou plusieurs hétéroatomes choisis parmi l'oxygène, le soufre ou l'azote, et pouvant être substituée par un ou plusieurs radicaux hydroxyle, alcoxy en C₁-C₆ ou amino, et pouvant comprendre une ou plusieurs fonctions carbonyle ;
• R₄, R₅ et R₆, pris séparément, représentent un radical alkyle en C₁-C₁₅ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical carbamoylalkyle en C₁-C₆ ; un radical trialkyl(C₁-C₆)silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl(C₁-C₆)carbonyle, carbamoyle ou alkyl(C₁-C₆)sulfonyle ; un radical alkyl(C₁-C₆)carboxyalkyle en C₁-C₆, un radical alkyl(C₁-C₆)carbonylalkyle en C₁-C₆ ; un radical N alkyl(C₁-C₆)carbamoylalkyle en C₁-C₆.
• R₄, R₅ et R₆ ensemble, deux à deux, forment, avec l'atome d'azote auxquels ils sont rattachés, un cycle saturé carboné à 4, 5, 6 ou 7 chaînons pouvant contenir un ou plusieurs hétéroatomes, le cycle cationique pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical carboxyle, un radical alkyl(C₁-C₆)carbonyle, un radical thio, un radical thioalkyle en C₁-C₆, un radical alkyl(C₁-C₆)thio, un radical amino, un radical amino mono- ou di-substitué par un radical alkyl(C₁-C₆), alkyl(C₁-C₆)carbonyle, carbamoyle ou alkyl(C₁-C₆)sulfonyle;
• R₇ représente un radical alkyle en C₁-C₆ ; un radical monohydroxyalkyle en C₁-C₆ un radical polyhydroxyalkyle en C₂-C₆ ; un radical aryle ; un radical benzyle ; un radical aminoalkyle en C₁-C₆ ; un radical aminoalkyle en C₁-C₆ dont l'amine est mono- ou di-subsitué par un radical alkyl(C₁-C₆), alkyl(C₁-C₆)carbonyle, carbamoyle ou alkyl(C₁-C₆)sulfonyle ; un radical carboxyalkyle en C₁-C₆; un radical carbamoylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• x est 0 ou 1,
- lorsque x = 0, alors le bras de liaison est rattaché à l'atome d'azote portant les radicaux R₄ à R₆,
- lorsque x = 1, alors deux des radicaux R₄ à R₆ forment conjointement avec l'atome d'azote auquel ils sont rattachés un cycle saturé à 4, 5, 6 ou 7 chaînons et D est lié à un atome de carbone du cycle saturé ;
• Y⁻ est un contre ion.

6. Dérivés selon la revendication 5 dans lesquels x est égal à 0, et R₄, R₅ et R₆ séparément sont choisis parmi un radical alkyle en C₁-C₆, un radical monohydroxyalkyle en C₁-C₄, un radical polyhydroxyalkyle en C₂-C₄, un radical alcoxy(C₁-C₆)alkyle en C₁-C₄, un radical carbamoylalkyle en C₁-C₆, un radical trialkyl(C₁-C₆)silanealkyle en C₁-C₆ , ou R₄ avec R₅ forment ensemble un cycle azétidine, pyrrolidine, pipéridine, pipérazine, morpholine, R₆ étant choisi dans ce cas parmi un radical alkyle en C₁-C₆ ; un radical monohydroxyalkyle en C₁-C₆ ; un radical polyhydroxyalkyle en C₂-C₆; un radical aminoalkyle en C₁-C₆, un radical aminoalkyle en C₁-C₆ dont l'amine est mono- ou di-substitué par un radical alkyl(C₁-C₆), alkyl(C₁-C₆)carbonyle, carbamoyle ou alkyl(C₁-C₆)sulfonyle ; un radical carbamoylalkyle en C₁-C₆ ; un radical trialkyl(C₁-C₆)silanealkyle en C₁-C₆ ; un radical alkyl(C₁-C₆)carboxyalkyle en C₁-C₆ ; un radical alkyl(C₁-C₆)carbonylalkyle en C₁-C₆ ; un radical N-alkyl(C₁-C₆)carbamylalkyle en C₁-C₆.

7. Dérivés selon la revendication 5 dans lesquels x est égal à 1, R₇ est choisi parmi un radical alkyle en C₁-C₆ ; un radical monohydroxyalkyle en C₁-C₆ ; un radical polyhydroxyalkyle en C₂-C₆ ; un radical aminoalkyle en C₁-C₆, un radical aminoalkyle en C₁-C₆ dont l'amine est mono ou disubstituée par un radical alkyl(C₁-C₆), alkyl(C₁-C₆)carbonyle, carbamoyle ou alkyl(C₁-C₆)sulfonyle ; un radical carbamylalkyle en C₁-C₆ ; un radical trialkyl(C₁-C₆)silanealkyle en C₁-C₆ ; un radical alkyl(C₁-C₆)carboxyalkyle en C₁-C₆ ; un alkyl(C₁-C₆)carbonylalkyle en C₁-C₆ ; un radical N-alkyl(C₁-C₆)carbamoylalkyle en C₁-C₆ ; R₄ avec R₅ ensemble forment un cycle azétidine pyrrolidine, pipéridine, pipérazine, morpholine, R₆ étant choisi dans ce cas parmi un radical alkyle en C₁-C₆ ; un radical monohydroxyalkyle en C₁-C₆ ; un radical polyhydroxyalkyle en C₂-C₆; un radical aminoalkyle en C₁-C₆, un radical aminoalkyle en C₁-C₆ dont l'amine est mono ou disubstituée par un radical alkyl(C₁-C₆), alkyl(C₁-C₆)carbonyle, carbamoyle ou alkyl(C₁-C₆)sulfonyle ; un radical carbamylalkyle en C₁-C₆ ; un radical trialkyl(C₁-C₆)silanealkyle en C₁-C₆ ; un radical alkyl(C₁-C₆)carboxyalkyle en C₁-C₆ ; un radical alkyl(C₁-C₆)carbonylalkyle en C₁-C₆ ; un radical N-alkyl(C₁-C₆)carbamylalkyle en C₁-C₆ .

8. Dérivés selon l'une quelconque des revendications 1 à 7 dans lesquels le radical onium Z est un trialkylammonium.

9. Dérivés selon l'une quelconque des revendications 1 à 8 dans lesquels D est une simple liaison ou une chaîne alkylène en C₁-C₈ pouvant être substituée.

10. Dérivés de paraphénylènediamine selon l'une quelconque des revendications 1 à 4 dans lesquels le radical onium Z correspond à la formule (III) 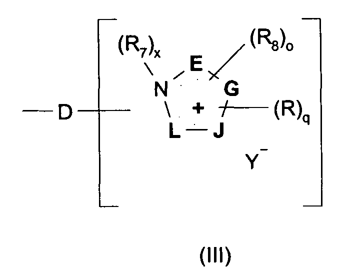 dans laquelle
• D est une liaison covalente ou une chaîne alkylène en C₁-C₁₄, linéaire ou ramifiée pouvant contenir un ou plusieurs hétéroatomes choisis parmi l'oxygène, le soufre ou l'azote, et pouvant être substituée par un ou plusieurs radicaux hydroxyle, alcoxy en C₁-C₆ ou amino, et pouvant comprendre une ou plusieurs fonctions carbonyle;
• les sommets E, G, J, L, identiques ou différents, représentent un atome de carbone, d'oxygène, de soufre ou d'azote pour former un cycle pyrrolique, pyrazolique, imidazolique, triazolique, oxazolique, isooxazolique, thiazolique, isothiazolique,
• q est un nombre entier compris entre 1 et 4 inclus ;
• o est un nombre entier compris entre 1et 3 inclus ;
• q+o est un nombre entier compris entre 2 et 4
• R, identiques ou différents, représentent un atome d'hydrogène, un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical carboxyle, un radical alkylcarbonyle en C₁-C₆, un radical thio, un radical thioalkyle en C₁-C₆, un radical alkyl(C₁-C₆)thio, un radical amino, un radical amino mono ou di substituée par un radical alkyl(C₁-C₆), alkyl(C₁-C₆)carbonyle, carbamoyle, alkyl(C₁-C₆)sulfonyle ; un radical monohydroxyalkyle en C₁-C₆ ou un radical polyhydroxyalkyle en C₂-C₆ ; étant entendu que les radicaux R sont portés par un atome de carbone,
• R₈ , identiques ou différents, représentent un atome d'hydrogène, un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamoylalkyle C₁-C₆, un radical alkyl(C₁-C₆)carboxyalkyle en C₁-C₆, un radical benzyle ; étant entendu que les radicaux R₈ sont portés par un azote,
• R₇ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est mono ou di substituée par un radical alkyl(C₁-C₆), alkyl(C₁-C₆)carbonyle, carbamoyle ou alkyl(C₁-C₆)sulfonyle ; un radical carboxyalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carboxyalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• x est 0 ou 1
- lorsque x = 0, le bras de liaison D est rattaché à l'atome d'azote,
- lorsque x = 1, le bras de liaison D est rattaché à l'un des sommets E, G, J ou L,
• Y⁻ est un contre-ion.

11. Dérivés selon la revendication 10 dans lesquels les sommets E, G, J et L forment un cycle pyrrolique, imidazolique, pyrazolique, oxazolique, thiazolique et triazolique.

12. Dérivés selon la revendication 11 dans lesquels les sommets E, G, J et L forment un cycle imidazolique.

13. Dérivés selon l'une quelconque des revendications 10 à 12 dans lesquels x est égal à 0, D est une simple liaison ou une chaîne alkylène en C₁-C₈ pouvant être substituée.

14. Dérivés de paraphénylènediamine selon l'une quelconque des revendications 1 à 4 dans lesquels le radical onium Z correspond à la formule (IV) 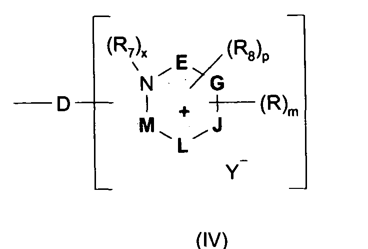 dans laquelle :
• D est une liaison covalente ou une chaîne alkylène en C₁-C₁₄, linéaire ou ramifiée pouvant être interrompue par un ou plusieurs hétéroatomes choisis parmi un atomes d'oxygène, de soufre ou d'azote, et pouvant être substituée par un ou plusieurs radicaux hydroxyle, alcoxy en C₁-C₆ ou amino, et pouvant comprendre une ou plusieurs fonctions carbonyle;
• les sommets E, G, J, L et M identiques ou différents, représentent un atome de carbone, d'oxygène, de soufre ou d'azote et forment un cycle choisi parmi les cycles pyridiniques, pyrimidiniques, pyraziniques, triaziniques et pyridaziniques,
• p est un nombre entier compris entre 1 et 3 inclus ;
• m est un nombre entier compris entre 1 et 5 inclus ;
• p+m est un nombre entier compris entre 2 et 5 ;
• R, identiques ou différents, représentent un atome d'hydrogène, un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical carboxyle, un radical alkylcarbonyle en C₁-C₆, un radical thio, un radical thioalkyle en C₁-C₆, un radical alkyl(C₁-C₆)thio, un radical amino, un radical amino substitué par un radical alkyl(C₁-C₆), alkyl(C₁-C₆)carbonyle, carbamoyle ou alkyl(C₁-C₆)sulfonyle ; un radical monohydroxyalkyle en C₁-C₆ ou un radical polyhydroxyalkyle en C₂-C₆ ; étant entendu que les radicaux R sont portés par un atome de carbone,
• R₈ , identiques ou différents, représentent un atome d'hydrogène, un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ; étant entendu que les radicaux R₈ sont portés par un azote,
• R₇ représente un radical alkyle en C₁-C₆ ; un radical monohydroxyalkyle en C₁-C₆ un radical polyhydroxyalkyle en C₂-C₆ ; un radical aryle ; un radical benzyle ; un radical aminoalkyle en C₁-C₆, un radical aminoalkyle en C₁-C₆ dont l'amine est mono- ou di- substituée par un radical alkyl(C₁-C₆), alkyl(C₁-C₆)carbonyle, carbamoyle ou alkyl(C₁-C₆)sulfonyle ; un radical carboxyalkyle en C₁-C₆; un radical carbamoylalkyle en C₁-C₆ ; un radical trifluoroalkyle en C₁-C₆ ; un radical trialkyl(C₁-C₆)silanealkyle en C₁-C₆; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carboxyalkyle en C₁-C₆ ; un radical N-alkyl(C₁-C₆)carbamoylalkyle en C₁-C₆ ; un radical N-alkyl(C₁-C₆)sulfonamidoalkyle en C₁-C₆ ;
• x est 0 ou 1
- lorsque x = 0, le bras de liaison D est rattaché à l'atome d'azote,
- lorsque x = 1, le bras de liaison D est rattaché à l'un des sommets E, G, J, L ou M,
• Y représente un contre ion.

15. Dérivés selon la revendication 14 dans lesquels les sommets E,G,J,L et M avec l'azote du cycle forment un cycle choisi parmi les cycles pyridiniques, pyrimidiniques.

16. Dérivés selon l'une quelconque des revendications 10 à 15 dans lesquels x est égal à 0 et R est choisi parmi un atome hydrogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical alkylcarbonyle en C₁-C₆, un radical amino, un radical amino mono- ou di-substitué par un radical alkyl(C₁-C₆), alkyl(C₁-C₆)carbonyle, carbamoyle ou alkyl(C₁-C₆)sulfonyle ; un radical monohydroxyalkyle en C₁-C₆ ou un radical polyhydroxyalkyle en C₂-C₆ et R₈ est choisi parmi un atome hydrogène, un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆.

17. Dérivés selon l'une quelconque des revendications 10 à 15 dans lequel x est égal à 1, R₇ est choisi parmi un radical alkyle en C₁-C₆ ; un radical monohydroxyalkyle en C₁-C₆ ; un radical polyhydroxyalkyle en C₂-C₆ un radical aminoalkyle en C₁-C₆, un radical aminoalkyle en C₁-C₆ dont l'amine est mono- ou di- substituée par un radical alkyl(C₁-C₆), alkyl(C₁-C₆)carbonyle, un radical carbamoyle ou un radical alkyl(C₁-C₆)sulfonyle ; un radical carbamoylalkyle en C₁-C₆ ; un radical trialkyl(C₁-C₆)silanealkyle en C₁-C₆ ; un radical alkyl(C₁-C₆)carbonylalkyle en C₁-C₆ ; un radical N-alkyl(C₁-C₆)carbamylalkyle en C₁-C₆ ; R est choisi parmi un atome hydrogène , un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical carbamoyle, un radical alkylcarbonyle en C₁-C₆, un radical amino, un radical amino mono- ou di- substitué par un radical alkyl(C₁-C₆), alkyl(C₁-C₆)carbonyle, carbamoyle ou alkyl(C₁-C₆)sulfonyle ; et R₈ est choisi parmi un atome hydrogène , un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamoylalkyle C₁-C₆.

18. Dérivés selon l'une quelconque des revendications 10 à 17 dans lesquels R et R₈ sont des atomes d'hydrogène ou des radicaux alkyles pouvant être substitués et R₇ est un radical alkyle pouvant être substitué

19. Dérivés selon l'une quelconque des revendications précédentes dans lesquels R₂ est un radical onium Z et R₃ est choisi parmi un atome d'hydrogène

20. Dérivés selon l'une quelconque des revendications 1 à 18 dans lesquels R₂ est un radical onium Z et R₃ est choisi parmi un radical hydroxyle, un radical amino, un radical amino mono- ou di- substituée par un radical alkyl(C₁-C₆) ; un radical alkyl(C₁-C₆)carbonyle, un radical carbamoyle ou un radical alkyl(C₁-C₆)sulfonyle

21. Dérivés selon l'une quelconque des revendications 1 à 18 dans lesquels R₃ est un radical onium Z et R₂ est choisi parmi un radical hydroxyalkyle en C₁-C₄, un radical carbamoyle, un radical mono ou dialkyl(C₁-C₄)carbamoyle, un radical carboxyle, un radical alkyl(C₁-C₄)oxycarbonyle, un radical aminoalkyle en C₁-C₄, un radical aminoalkyle en C₁-C₄ dont l'amine est mono ou disubstituée par un radical alkyle en C₁-C₄.

22. Dérivés selon l'une quelconque des revendications 1 à 18 dans lesquels R₂ et R₃ sont des radicaux oniums Z.

23. Dérivés nitrophénylène de formule (I') 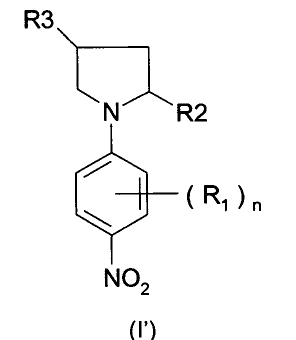 dans laquelle R₁, R₂, R₃ et n sont tels que définis dans l'une quelconque des revendications 1 à 22.

24. Composition tinctoriale comprenant à titre de base d'oxydation au moins un dérivé paraphénylènediamine de formule (I), tel que défini selon l'une quelconque des revendications 1 à 22.

25. Composition selon la revendication 24 comprenant de plus un coupleur choisi parmi les méta-phénylènediamines, les méta-aminophénols, les méta-diphénols, les coupleurs naphtaléniques, les coupleurs hétérocycliques et leurs sels d'addition

26. Composition selon l'une quelconque des revendications 24 ou 25 comprenant une base d'oxydation additionnelle autre que les bases d'oxydation de formule (I) choisie parmi les para-phénylènediamines, les bis-phénylalkylènediamines, les para-aminophénols, les ortho-aminophénols, les bases hétérocycliques et leurs sels d'addition.

27. Composition selon l'une quelconque des revendications 24 à 26 dans laquelle la quantité de chacune des bases d'oxydation est comprise entre 0,001 à 10 % en poids environ du poids total de la composition tinctoriale.

28. Composition selon la revendication 27 dans laquelle la quantité de chacun des coupleurs est comprise entre 0,001 et 10 % en poids environ du poids total de la composition tinctoriale.

29. Composition selon l'une quelconque des revendications 24 à 28 comprenant de plus un milieu cosmétique approprié à la teinture des fibres kératiniques humaines.

30. Procédé de teinture d'oxydation des fibres kératiniques, **caractérisé en ce qu'**on applique sur les fibres une composition tinctoriale telle que définie dans l'une quelconque des revendications 24 à 29 en présence d'un agent oxydant pendant un temps suffisant pour développer la coloration désirée.

31. Procédé selon la revendication 30 dans lequel l'agent oxydant est choisi parmi le peroxyde d'hydrogène, le peroxyde d'urée, les bromates de métaux alcalins, les persels, les peracides et les enzymes oxydases.

32. Dispositif à plusieurs compartiments dans lequel un premier compartiment contient une composition tinctoriale telle que définie à l'une quelconque des revendications 24 à 29 et un deuxième compartiment contient un agent oxydant.

33. Utilisation de la composition définie aux revendications 24 à 29 pour la teinture de fibres kératiniques.
